# EUROPEAN PATENT SPECIFICATION

(11) **EP 2 488 226 B1**
(45) Date of publication and mention of the grant of the patent: **20.11.2013**
(21) Application number: 10757207.5
(22) Date of filing: 24.09.2010
(51) Int. Cl.: A61M 1/10, A61M 1/16, F04B 43/06

(54) **MEMBRANE PUMP SYSTEM**
MEMBRANPUMPENSYSTEM
SYSTÈME À POMPE À MEMBRANE

(30) Priority: 15.10.2009 SE 0901326; 19.10.2009 US 252741 P
(43) Date of publication of application: 22.08.2012
(73) Proprietor: Gambro Lundia AB, 220 10 Lund (SE)
(72) Inventor: NILSSON, Eddie, S-243 32 Höör (SE); JÖNSSON, Lennart, S-237 42 Bjärred (SE); HOLMER, Mattias, S-224 73 Lund (SE)
(74) Representative: Bornegard, Annette
(86) International application number: PCT/EP2010/064118
(87) International publication number: WO 2011/045167

(56) References cited:
- WO-A2-2009/127624
- GB-A- 2 190 657
- US-A- 4 222 127
- US-A- 4 250 872
- US-A1- 2009 099 498

## Description

### Technical field of the present invention

The present invention generally relates to a membrane pump i.e. a positive displacement pump that uses a combination of the reciprocating action of a membrane and suitable non-return check valves to pump a fluid. The present invention is e.g. applicable to a membrane pump for use in a blood treatment apparatus.

### Background of the invention

WO 2008/106440 discloses a pod pump that is incorporated into a fluid control or pump cassette. A membrane separates the central cavity of the pod pump into two chambers, a pumping chamber and an actuation chamber. The pumping chamber receives the fluid to be pumped and the actuation chamber receives a control gas that pneumatically actuates the pump. Pneumatic pressure is provided through a pneumatic port to either force, with positive gas pressure, the membrane towards one wall of the pod pump cavity to minimize the pumping chamber's volume or to draw, with negative gas pressure, the membrane towards the other wall of the pod pump cavity to maximize the pumping chamber's volume. Further, WO 2008/106440 discloses a membrane that has a variable cross-sectional thickness, e.g. the membrane may have its thickest cross-sectional area closest to its center. On the fluid side, a groove may be present on the chamber wall. The groove acts to prevent folds in the membrane from trapping fluid in the chamber when emptying. Further, it allows for the fluid to continue flowing through the pod pumps after the membranes reaches the end of stroke.

### Summary of the invention

It is an object of the invention to provide an alternative and improved membrane pump for a pumping system.

It is a further object of the invention to minimize a risk of an air bubble being trapped in the pumping chamber of a membrane pump.

It is a further object of the invention to establish a pressure signal that is indicative of end of stroke of a flexible member in a membrane pump.

It is a further object of the invention to provide passage of a certain amount of flow, referred to as base flow Q when a flexible member in a membrane pump has reached its end position.

It is a further object of the invention to improve emptying a first accumulation container of a membrane pump from blood.

This and other objects, which will appear from the description below, are at least achieved by means of membrane pump and a blood treatment arrangement according to the independent claims, embodiments thereof being defined by the dependent claims.

A first aspect of the invention is a membrane pump comprising a pumping chamber and a flexible member separating the pumping chamber into a first accumulation container and a second accumulation container. The flexible member is configured to be movable within the pumping chamber so as to vary a volume relationship between the first and second accumulation container. The second accumulation container is configured to receive an amount of working fluid to act on the flexible member and thus, in use, pump a first fluid from the first accumulation container. The first and the second accumulation container, each has an inlet opening for inlet of the respective fluid and an outlet opening for outlet of the respective fluid, the inlet opening being arranged on one side of the accumulation container and the outlet opening being arranged on the opposite side of the accumulation container. The flexible member is configured to show, when arranged in the pumping chamber and seen in a cross section along a plane extending through a pair of the inlet and outlet openings of the accumulation container, a thickness that is increased in a downstream direction.

In one embodiment of the first aspect of the invention the thickness of the flexible member is increased in a continuous manner.

In another embodiment of the first aspect of the invention the thickness of the flexible member is increased by at least 50%.

In another embodiment of the first aspect of the invention the flexible member has a dome shape.

In a further embodiment of the first aspect of the invention the dome shaped portion is formed as a section of a spherical object having an inner radius and an outer radius where the central point for the outer radius is offset with respect to the central point of the inner radius.

In still a further embodiment of the first aspect of the invention a rigid wall of the second accumulation container is provided with at least one groove extending between the inlet and the outlet where the at least one groove has a cross sectional area that is less than the cross sectional area of the inlet opening.

In a further embodiment of the first aspect of the invention the at least one groove has a cross sectional area that is substantially 1/10 of the cross sectional area of the inlet opening.

In a further embodiment of the first aspect of the invention the at least one groove has a width and a depth where the ratio between the width and the depth is <1. Alternatively the ratio is substantially 1/1,5.

In a further embodiment the first aspect of the invention a rigid wall of the second accumulation container is provided with a plurality of grooves extending between an area around the inlet and the outlet such that, upon closure of the inlet by means of the flexible member, a flow path disconnected from the inlet is provided.

In another embodiment of the first aspect of the invention the flexible member is arranged dividing the first and the second accumulation containers along a substantially vertical plane.

A second aspect of the invention is a blood treatment apparatus, comprising a blood treatment unit configured to receive untreated blood and fresh blood treatment fluid, and emit treated blood and used blood treatment fluid. The blood treatment unit being connectable to at least one fluid pump configured to pass blood treatment fluid through the blood treatment unit, and at least two blood pumps configured to extract untreated blood from a blood source pass extracted blood through the blood treatment unit and deliver treated blood to a target vessel. At least one of the at least one fluid pump is configured to control the operation of the blood pumps via the blood treatment fluid, and the blood pumps are integrated into a joint apparatus element comprising the blood treatment unit. The blood pumps of a type according to the first aspect of the invention.

In an embodiment of the second aspect of the invention each of the at least one blood pump is configured to be in fluid connection with the treatment unit throughout an entire treatment of an amount of blood from the blood source.

In another embodiment of the second aspect invention a first blood conduit is configured to be connected to the blood source, a second blood conduit configured to be connected to the target vessel, and at least one needle connector is configured to connect the first and second blood conduits with at least one needle.

In a further embodiment of the second aspect invention the joint apparatus element comprises a body module having an essentially cylindrical outline with a central length axis, and at least one of the at least one blood pump in the joint apparatus element is arranged on a side surface of the blood treatment unit such that the at least one blood pump is arranged essentially asymmetrically with respect to the central length axis.

Still other objects, features, advantages and embodiments of the present invention will become apparent from the following detailed description when taken in conjunction with the claims and drawings.

### BRIEF DESCRIPTION OF THE DRAWINGS

Figure 1 shows a block diagram over a blood treatment apparatus according to a first embodiment of the invention.
Figure 2 shows a block diagram over a blood treatment apparatus according to a second embodiment of the invention.
Figures 3a-f show perspective views of the proposed joint apparatus element according to a first embodiment of the invention.
Figure 4a, 4b show schematically in a cross sectional view A-A in Figure 3f a membrane pump according to one embodiment of the present invention.
Figure 5 shows schematically in a cross sectional view a flexible member according to one embodiment of the present invention.
Figure 6a shows in a cross sectional view C-C in Figure 4b a schematic illustration of grooves in a rigid wall of a blood side of the membrane pump.
Figure 6b shows in a cross sectional view D-D in Figure 4a a schematic illustration of grooves in a rigid wall of a fluid side of the membrane pump.
Figure 6c shows is a schematic illustration of a cross sectional area of a groove in a rigid wall of the membrane pump.

### DESCRIPTION OF EMBODIMENTS OF THE INVENTION

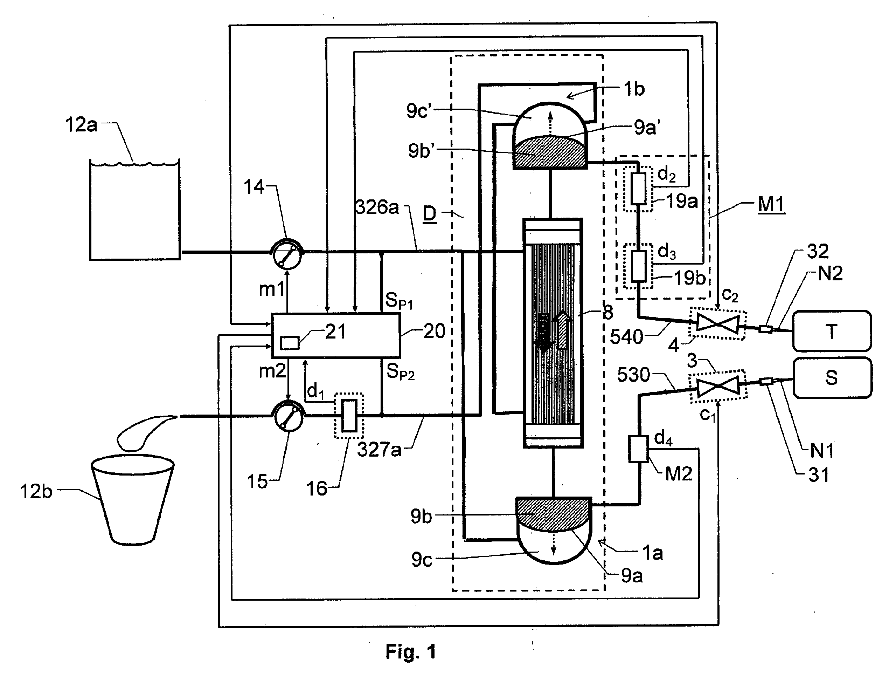

Figure 1, shows a block diagram over a blood treatment apparatus (e.g. a dialysis apparatus) according to a first embodiment of the invention.

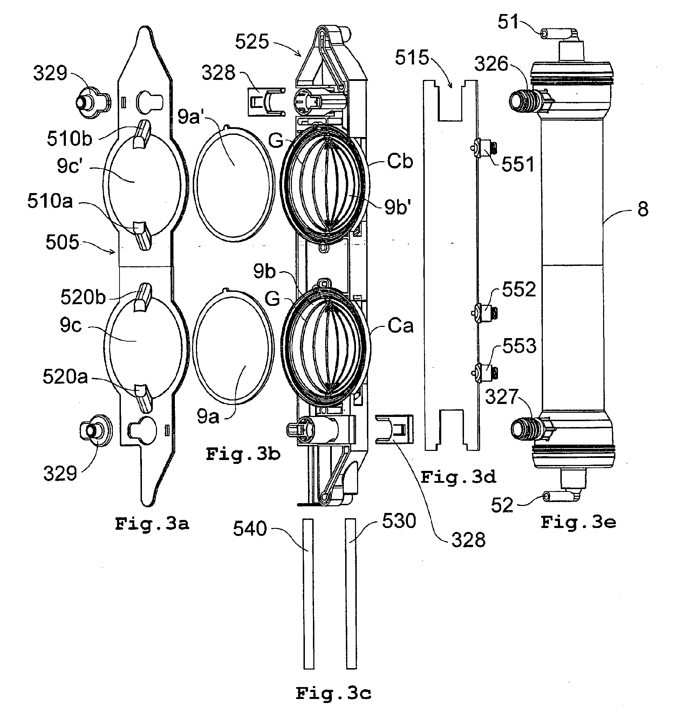

The apparatus comprises a fluid circuit that includes a blood treatment unit 8 (typically represented by a dialyzer), a first and a second fluid pump 14, 15 and a first and a second blood pump 1a, 1b. The fluid pumps 14, 15 are configured to pass a blood treatment fluid (e.g. dialysis fluid) from a fluid container 12a through the blood treatment unit 8, via the first and the second blood pump 1a, 1b. The first blood pump 1a is further configured to extract untreated blood from a blood source S and pass the extracted blood through the blood treatment unit 8. The second blood pump 1b is configured to receive treated blood from the blood treatment unit 8 and deliver the treated blood to a target vessel T. The fluid pumps 14, 15 are configured to control the operation of the first and the second blood pumps 1a, 1b via the blood treatment fluid. Moreover, the blood pumps 1a, 1b are integrated into a joint apparatus element D wherein the blood treatment unit 8 is connected to a backbone structure (see Figure 3a-3f).

In order to control the operation of the blood pumps 1a, 1b by means of the fluid pumps 14, 15, the blood pumps 1a, 1b each has a pumping chamber, which is separated into first and second accumulation containers 9b, 9c; 9b', 9c' respectively. Further, a flexible member 9a,- 9a'- (e.g. in the form of a soft/elastic membrane) constitutes a separation wall between the first and second accumulation containers 9b, 9c; 9b', 9c'.

The flexible member 9a, 9a' is movable within the pumping chamber so as to vary a volume relationship between the first and second accumulation containers 9b, 9c; 9b', 9c'. In one embodiment a stoke volume of the pumping chamber may be in the range of 15-50 ml. In an alternative embodiment the stroke volume may be in the range of 5-20 ml.

First and second motoric signals m1 and m2 from a control unit 20 control the operation of the fluid pumps 14, 15 respectively. Specifically, during a first phase, the first accumulation container 9b of the first blood pump 1a is charged with untreated blood from the blood source S and the first accumulation container 9b' of the second blood pump 1b is charged with treated blood from the treatment unit 8. In parallel therewith, fresh blood treatment fluid F is discharged from the second accumulation chamber 9c of the first blood pump 1a and through the treatment unit 8 to the second accumulation container 9c' of the second blood pump 1b and from there to a waste compartment 12b by means of the second fluid pump 15. Then, during a second phase, the first fluid pump 14 pumps fresh blood treatment fluid F from the fluid container 12a, through the blood treatment unit 8 and into the second accumulation container 9c' of the second blood pump 1b as well as to the second accumulation container 9c of the first blood pump 1a. In parallel therewith, blood is pushed through the blood treatment unit 8 and via the first accumulation container 9a' of the second blood pump to the target vessel T. Hence, blood and blood treatment fluid pass simultaneously through the unit 8.

To enable a desired operation of the blood treatment apparatus, the apparatus includes first and second blood valve means 3 and 4 respectively. The first blood valve means 3 is configured to control the extraction of un-treated blood from the blood source S· via a needle N1, and the second blood valve means 4 is configured to control the return of treated blood to the target vessel T, via the needle N2. The blood valve means 3 and 4 need to be activated alternately, such that the first blood valve means 3 is open while the second blood valve 4 is closed, and vice versa.

The first and the second fluid pumps 14, 15 may be operated in such a manner that, in addition to the treatment fluid flow F, a base flow Q is provided. The base flow Q is passed through the blood treatment unit 8 during both the first and second phases of the cyclic process. The base flow Q is independent of the flow of untreated blood extracted from the blood source, S. The base flow Q may be used together with one or more flow control means (not shown) to control the blood pumps 1a, 1b to operate in a synchronized manner by securing the same flow of blood treatment fluid to and from the respective second accumulation container 9c, 9c'. Furtheron, the base flow Q may be used together with the one or more flow control means to control the transmembrane flow.

In the embodiment of the invention illustrated in Figure 1, first and second pressure parameters are measured via pressure sensor signals S_{P1}, S_{P2}, which are registered on a conduit configured to pass fresh blood treatment fluid F from the fluid container 12a to the blood treatment unit 8 and on a conduit configured to pass used blood treatment fluid F from the blood treatment unit 8 to the waste compartment 12b. For reasons of simplicity, we here assume that a pressure measuring unit is included in a control unit 20. In any case, the pressure measuring unit does not come into contact with the blood. Instead, the blood pressure is measured via the blood treatment fluid, which due to the contact with the respective flexible member 9a; 9a' when moved between its respective end position has a pressure level equal to that of the blood.

Specifically, the first pressure parameter represents a first pressure level of the untreated blood extracted from the blood source S, and the second pressure parameter represents a second pressure level of the treated blood being returned to the target vessel T. Additionally, the first and second pressure parameters are registered at different points in time. Therefore, one single pressure measuring means is sufficient to determine both pressure parameters.

Typically, in the blood treatment unit 8, the blood treatment fluid F pass through a large number of hollow fibers made from semi-permeable membrane, and dialysis takes place over the semi-permeable membrane.

The blood treatment unit 8 has a fluid outlet conduit, which is configured to discharge used blood treatment fluid F from the apparatus during the first phase of the cyclic process. The discharged blood treatment fluid F optionally continues into the waste compartment 12b, or down the drain.

It is further advantageous if the control unit 20, in response to the pressure sensor signal S_{P} (expressing the first and second pressure parameters S_{P2}, S_{P1}), is configured to control the first and second blood valve means 3 and 4 such that the cyclic process is effected. Optionally, the control unit 20, in turn, includes, or is associated with; a memory means 21 storing computer software for controlling the control unit 20 to effect the above-described procedure.

It is preferable if a blood leak detector 16 is arranged on the fluid conduit between the blood treatment unit 8 and the second fluid pump 15. Thereby any malfunction of the apparatus resulting in that blood enters into the blood treatment fluid path (e.g. due to a leaking blood pump 1a, 1b or leakage in the blood treatment unit 8) may be reported to the control unit 20 by the blood leak detector 16 via fist detection signal d₁. In response to the first detection signal d₁, the control unit 20 may generate an alarm, such that appropriate corrective actions may be taken.

In a start up phase (i.e. prior to initiating the above-mentioned cyclic process) the fluid circuit comprising fluid lines 326a, 327a, first and second blood pumps 1a, 1b and blood treatment unit 8 may be filled (or more precisely filled such that superfluous fluid rinses the circuit) with fresh blood treatment fluid (e.g. dialysis fluid) from the fluid container 12a. The filling of the fluid causes any air in the fluid circuit to be pushed back into the waste compartment 12b (or drain) where it is vented. Correspondingly, the first needle connection 31 and/or conduit 530 may be connected to a saline solution (or other appropriate fluid) to fill and rinse, and thus eliminate any gas bubbles in the blood circuit. This process of filling and rinsing the apparatus is often referred to as priming.

In one embodiment the first and the second blood pumps 1a, 1b are configured to be in fluid connection with the treatment unit 8 throughout an entire blood treatment session. Thus it is provided for a continuous blood flow through blood treatment unit 8 throughout the entire treatment although the blood flow may vary during certain periods, e.g. in connection with a transition between the first phase and the second phase. This means that a blood side of the apparatus in which the first and second blood pumps 1a, 1b are included is filled with blood until the treatment session is completed. Consequently, the blood may be treated/cleaned efficiently.

In a further embodiment the first fluid pump 14 is configured to be in fluid connection with the blood treatment unit 8 throughout an entire treatment session I.e. it is provided for a continuous blood treatment fluid flow throughout the entire treatment session although the blood treatment fluid flow may be varying during certain periods. This means that a fluid side of the apparatus in which the fluid pumps 14 and 15 are included is completely filled with blood treatment fluid F until the treatment session is completed. Again, this vouches for an efficient treatment/cleaning of the blood.

According to one embodiment of the invention, a first blood conduit 530 is configured to be connected to the blood source, S for receiving untreated blood, and a second blood conduit 540 is configured to be connected to the target vessel, T for delivering treated blood. A first and a second needle connector 31, 32 is configured to receive each of the first and second blood conduits 530 and 540 respectively.

The second blood conduit 540 may include a primary safeguard module M1, which is configured to check at least one quality parameter of the treated blood delivered to the target vessel T. For example, an air bubble detector 19a in the primary safeguard module M1 can be adapted to detect any undesired gas bubbles in the treated blood being delivered to the target vessel T, and in case a threshold value is exceeded, the air bubble detector 19a may deliver a data signal d₂ to the control unit 20. In response thereto, the control unit 20, in turn, optionally causes an appropriate alarm to be generated.

Alternatively, or as a complement to the above, the primary safeguard module M1 may include a fluid detector means 19b, which is adapted to detect if air, priming fluid or blood is passed through the apparatus. Hence, during the priming period, the fluid detector means 19b may detect when air fluid is substituted by priming fluid and when priming fluid is substituted with blood fluid. In response thereto, the fluid detector means 19b is configured to deliver a data signal d₃ to the control unit 20. The control unit 20, in turn, is optionally configured to cause, trigger or indicate performance of an appropriate action. In the same manner the same or a corresponding fluid detector means 19b is configured to detect when, during a blood restitution phase when treated blood is delivered to the target vessel T to end the treatment, blood fluid in the blood circuit is substituted by air fluid or a liquid (e.g. saline solution).

Furthermore, the first blood conduit 530 optionally includes a secondary safeguard module M2, which is configured to check at least one quality parameter of the untreated blood received from the blood source S, for instance detect the presence of gas bubbles and/or priming fluid. This renders it possible for the control unit 20 to cause generation of an alarm in response to a data signal d₄ from the secondary safeguard module M2, if the untreated blood is of inferior quality (e.g. due to the presence of gas bubbles caused by leakage). However, more important, if the apparatus unintentionally comes to pass blood in the opposite direction (i.e. back into the blood source S via the valve means 3), the secondary safeguard module M2 may detect unwanted substances (e.g. represented by gas bubbles) in this blood, and prevent these from reaching the blood source S.

In the embodiment shown in Figure 1 the second accumulation container 9c' of the second blood pump 1b is provided with an inlet for the blood treatment fluid that is separate from an outlet for the blood treatment fluid.

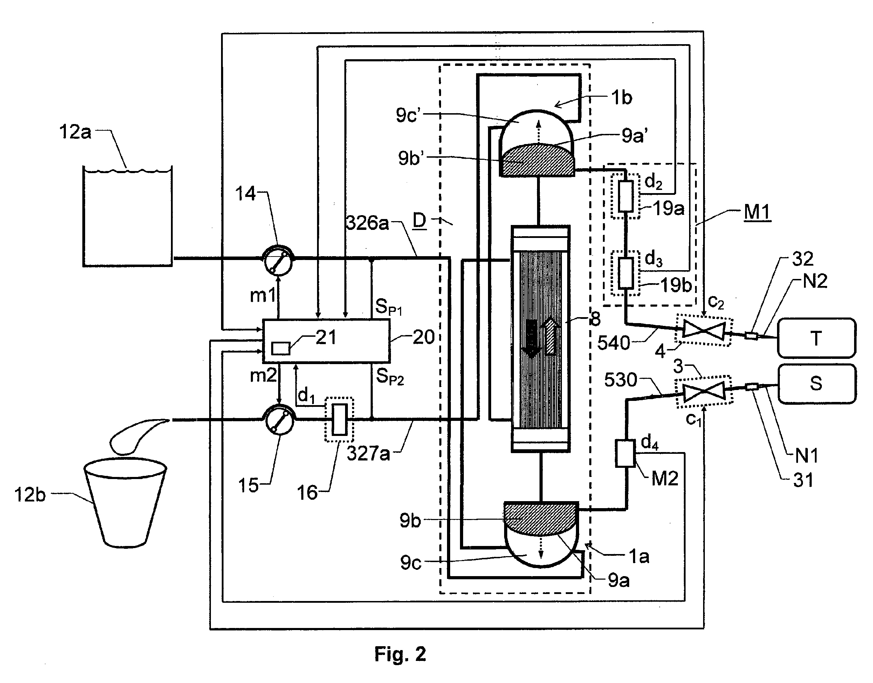

Figure 2 shows a block diagram over an apparatus similar to the one shown in Figure 1, however in this apparatus also the second accumulation container 9c of the first blood pump 1a is provided with an outlet separate from the inlet.

One advantage with separating the outlet for blood treatment fluid F from the inlet for blood treatment fluid F is that it is possible to create a repeatable behavior of the flow of blood treatment fluid F in to and out from the accumulation container 9c; 9c' The repeatable behavior is further improved if a suitable design of the flexible member 9a; 9a' is provided. The flexible member 9a; 9a' according to the present invention is more rigid in the area of the outlet than in the area of the inlet. The reinforced flexible member 9a; 9a' provides for closing the inlet before the outlet and thereby for the possibility to monitor a pressure signal indicative of when the flexible member 9a; 9a' in the blood pump 1a; 1b reaches its end position, end of stroke.

More specifically, a pressure peak is identifiable when the inlet for blood treatment fluid is closed. Thus upon end of stroke by the first flexible member 9a of the first blood pump 1a a first pressure measuring means (not shown) registers a pressure peak and emits a first pressure signal S_{P1}, i.e. a pressure signal which is indicative of end of stroke of the first flexible membrane 9a. Such an indication of end of stroke provides for simplified synchronization of the apparatus. Correspondingly, upon end of stroke by the second flexible member 9a' of the second blood pump 1b a second pressure measuring means (not shown) registers a pressure peak and emits a second pressure signal S_{P2}, i.e. a pressure signal which is indicative of end of stroke of the second flexible member 9a'. By synchronized, is meant that the respective flexible member 9a and 9a'of the respective first and second blood pump 1a and 1b reach their respective end position simultaneously.

According to one embodiment, the first and the second blood pumps 1a and 1b are connected with the blood treatment unit into a joint apparatus D. This is advantageous in a self-care environment because the user/patient may thereby mount and discard the entire joint apparatus element D before and after completion of the use in a treatment. This is desirable both from a handling and safety point-of-view. In some settings a disposable of this type is configured to be used for additional treatments and discarded when the treatment requirements are no longer fulfilled.

Figures 3a to 3f show perspective views of the proposed joint apparatus element according to a first design alternative, which is configured to be implemented with the apparatus illustrated in Figures 1 and 2.

The joint apparatus element D includes a body module that has an essentially cylindrical outline with a central length axis. The blood pumps 1a and 1b are arranged on a side surface of the blood treatment unit 8, such that the at least one blood pump 1a and 1b are located essentially asymmetrically with respect to the central length axis of the body module.

Figure 3a-3e shows the joint apparatus D in a disassembled state. More specifically, Figure 3a shows a first lid element 505; Figure 3b shows the flexible members 9a and 9a' of the first and second blood pumps 1a and 1b respectively; Figure 3c shows a backbone structure 525 and conduits 530 and 540 configured to receive and deliver blood respectively; Figure 3d shows a second lid element 515 configured to seal the backbone structure 525; and Figure 3e shows the blood treatment unit 8 and its fluid connections 326, 327 for connection with the fluid lines 326a and 327a and a blood outlet 51 and a blood inlet 52 respectively.

The first lid element 505 shows a first conduit 510a configured to pass blood treatment fluid F into the second accumulation container 9c' of the second blood pump 1b, and a second conduit 510b configured to pass blood treatment fluid F out from the second accumulation container 9c' of the second blood pump 1b. Correspondingly, the lid 505 also shows a first conduit 520a configured to pass blood treatment fluid F into the second accumulation container 9c of the first blood pump 1a, and a second conduit 520b configured to pass blood treatment fluid F out from the second accumulation container 9c of the first blood pump 1a.

In addition to blood and fluid channels, the backbone structure 525 includes inter alia a first pumping chamber Ca in which the flexible member 9a of the first blood pump 1a is mounted, and a second pumping chamber Cb in which the flexible member 9a' of the second blood pump 1b is mounted.

Figure 3c shows grooves G in the first accumulation container 9b; 9b' configured to hold blood B. Corresponding grooves G also exist in the second accumulation container 9c, 9c', configured to hold blood treatment fluid F. One purpose of the grooves G is to prevent the flexible member 9a, 9a' to be sucked into a locked position against the inner walls of the respective accumulation container 9b, 9c; 9b', 9c', in the end positions for the flexible member 9a; 9a'. Another purpose of these is to form a fluid path also when the flexible member 9a; 9a' has reached its end position to allow passage of the base flow Q. Further purposes of the grooves G are to facilitate emptying of fluids e.g. air, blood or blood treatment fluid, from the accumulation containers 9b, 9c; 9b', 9c'.

Further, in Figure 3a is shown a lid element 329 and in Figure 3b is shown securing plates 328 which are elements for fixing the first and the second fluid connections 326, 327 to fluid lines 326a, 327a in the assembled joint apparatus element D.

In an alternative embodiment (not shown) the backbone structure 525 and the blood treatment unit 8 are arranged spaced apart and connected via the fluid lines 326a, 327a.

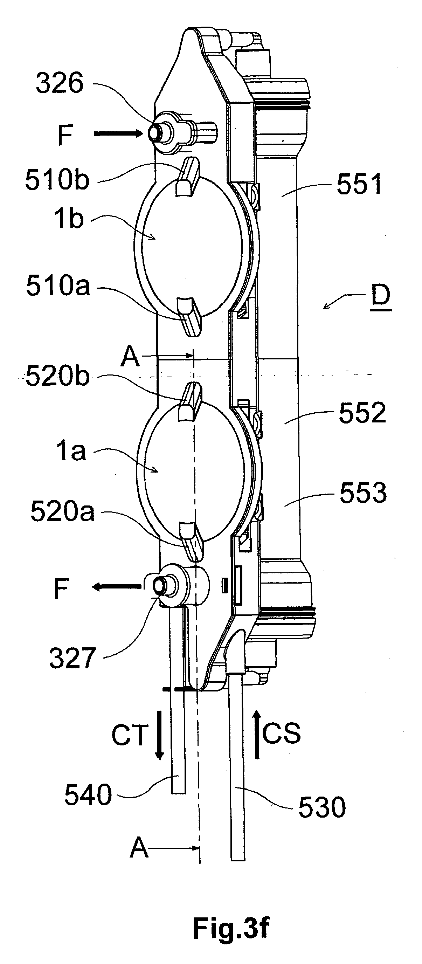

Figure 3f shows the joint apparatus element D according to Figure 3a-3e, in an assembled state. For instance, is shown the first and second blood pumps 1a and 1b respectively, a first fluid connection 326 to fluid line 326a configured to receive a flow of fresh blood treatment fluid F, a second fluid connection 327 to fluid line 327a configured to output a flow of used blood treatment fluid F, the conduit 530 configured to receive a flow CS of untreated blood from the blood source S, and the conduit 540 configured to deliver a flow CT of treated blood to the target vessel T. Additionally, Figure 3f embodies a sent of ports 551, 552 and 553, which may be used for auxiliary purposes, such as infusion of anticoagulant substances and/or drugs and/or for taking blood samples.

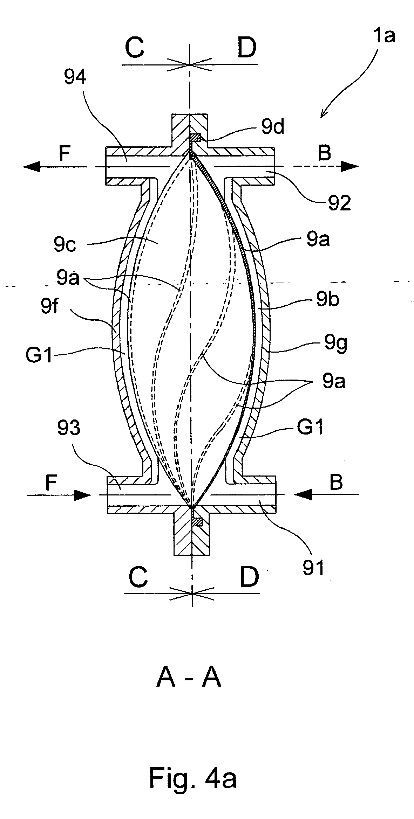

Figure 4a shows one embodiment of a membrane pump configured for acting as at least one of the above described blood pumps 1a, 1b.

The blood pump comprises, as described above in connection with the first blood pump 1a, a pumping chamber which is separated into first and second accumulation containers 9b, 9c by a flexible member 9a. The flexible member 9a is movable in the pumping chamber so as to vary a volume relationship between the first and second accumulation containers 9b and 9c. Further the first accumulation container 9b is configured to receive an amount of untreated blood B via a first inlet 91, the blood being delivered from a blood source S (see Figure 1 or 2), e.g. a bag or a patient. The blood exits the first accumulation container 9b via a first outlet 92. The second accumulation container 9c is configured to receive an amount of fresh blood treatment fluid F via a second inlet 93, the blood treatment fluid F being delivered from a blood treatment fluid container 12a (see Figure 1 or 2), e.g. a bag. The fluid exits the second accumulation container 9c via a second outlet 94. Optionally a base flow Q, as described above, is added to the flow of blood treatment fluid F.

In the embodiment of the invention shown in Figure 4a the first inlet 91 is separate from the first outlet 92 and the second inlet 93 is separate from the second outlet 94. The first and the second outlets 92, 94 are arranged downstream the first and the second inlets 91, 93 in the respective accumulation chamber 9b, 9c. The second inlet 93 is configured to be connected with the first conduit 520a shown in Figure 3f and the second outlet 94 is configured to be connected with the second conduit 520b shown in Figure 3f.

In Figure 4a the flexible member 9a is shown in a position where the second accumulation container 9c is filled with a blood treatment fluid F and the first accumulation container 9b is emptied of blood B. The flexible member 9a is, by broken line, indicated in multiple positions during filling of the first accumulation container 9b until the flexible member 9a is rested against the stiff or rigid wall 9f of the second accumulation container 9c.

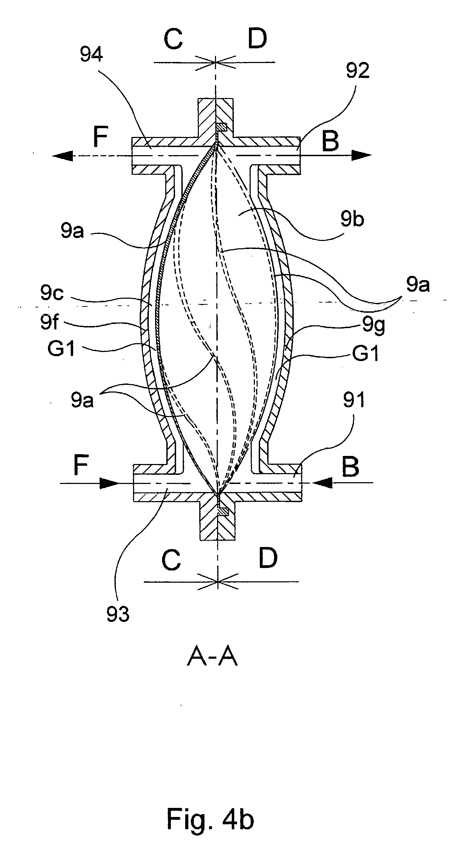

In Figure 4b the flexible member 9a is shown in a position where the first accumulation container 9b is filled with a blood B and the second accumulation container 9c is emptied of blood treatment fluid F. The flexible member 9a is, by broken line, indicated in multiple positions during filling of the second accumulation container 9c until the flexible member 9a is rested against the stiff or rigid wall 9g of the first accumulation container 9b.

The flexible member 9a has a predefined orientation in the pumping chamber such that it is arranged with a reinforced portion in the area of the respective outlet openings 92, 94, i.e. in a downstream direction of the pumping chamber. Since the flexible member 9a is reinforced in the area of the respective outlet openings 92, 94 and thereby more stiff in this area compared with its properties in the area of the inlet openings 91, 93, it will, during e.g. a blood filling phase (comparable with blood extraction phase at a system level), see Figure 4a, close the outlet opening 94 of the second accumulation container 9c only after having closed the inlet opening 93 thereof. Also the flexible member 9a will, during a blood emptying phase (comparable with a blood delivery phase at a system level), see Figure 4b, open the inlet opening 93 of the second accumulation container 9c before opening the outlet opening 92 thereof. Thus, the flexible member 9a according to the invention will minimize the risk for any air bubbles and any liquid being trapped in the accumulation container 9b, 9c upon end of stroke of the flexible member 9a, i.e. when the flexible member 9a is rested against the rigid wall 9f, 9g of the accumulation container 9b, 9c.

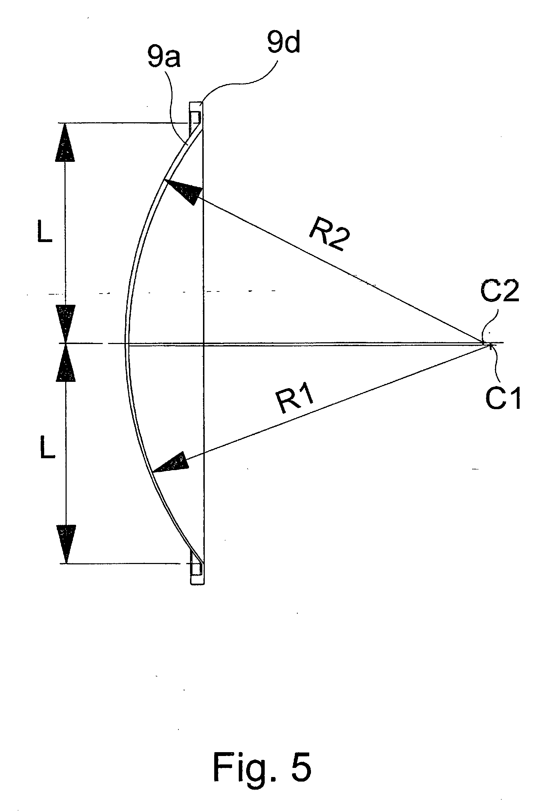

Figure 5 shows in greater detail the flexible member 9a shown in Figure 4a, 4b. The flexible member 9a is preformed with a dome-shape in a molding process. The dome-shape and the downstream increased thickness of the flexible member 9a may in this embodiment be described as a section of a spherical object having an inner radius R1 and an outer radius R2 where R1>R2 and where a central point C2 of the outer radius R2 is slightly offset with respect to a central point C1 of the inner radius R1 such that the thickness of the sphere increases from, as shown in Figure 5, a lower end of the flexible member 9a to an upper end of the flexible member 9a when seen in a cross sectional plane that is parallel with the direction in which the flexible member 9a is arranged to flex or move between its respective end positions. The flexible member 9a is provided with a rim 9d around its circumference for mounting between the rigid walls of the first and the second accumulation containers 9b, 9c.

It is realized that the form of the flexible member 9a; 9a' may be chosen from a large variety of geometries as long as it is combined with a pumping chamber having a suitable and corresponding geometry. E.g. the dome shaped form may occupy only a portion of the flexible member 9a; 9a' or the dome shaped form may be truncated.

As described above, the flexible member 9a shown in Figure 5 is configured to be arranged with a reinforced portion in a downstream direction of a pumping chamber. The flexible member 9a is configured to show, at least when arranged in the pumping chamber and seen in a cross section along a plane extending through a pair of the inlet an outlet openings 91, 92; 93, 94 of an accumulation container 9b; 9c, a thickness that increases in the downstream direction of the pumping chamber.

In the embodiment in Figure 5 the thickness is increased in a continuous manner. In an alternative embodiment (not shown) the thickness is increased in a stepwise manner.

Hence, the flexible member is less flexible in the area of the first and the second outlet 92, 94, than at the first and second inlet 91, 93. This results in an unsymmetrical flexing of the member 9a if compared between the filling and the emptying phase. This permits controlled/repeatable fluid flow into and out of the first and the second accumulation chamber 9b, 9c respectively. It further ensures that the first and the second outlet openings 92, 94 are not closed by the flexible member 9a before the accumulation chamber 9b, 9c is completely empty. The wall thickness of the flexible member 9a is in a downstream direction increased by at least 50%. In one embodiment of the invention the wall thickness is increased from 0,4 to 1 mm in the downstream direction.

In use in an embodiment of the present invention the pump chamber is arranged such that the flexible member 9a has a substantially vertical direction, i.e. the membrane is arranged dividing the accumulation containers 9b, 9c, 9b', 9c' along a substantially vertical plane. Further, the first and the second inlet openings 91, 93 are arranged in the lower part of the pump chamber. This embodiment ensures that any air in e.g. the first accumulation container 9b is pushed out upon emptying of the first accumulation container 9b. This is especially desirable during a priming phase when the extracorporeal circuit is flushed and filled with a priming fluid. It is also a desire during treatment to ensure no airbubbles are trapped in the first accumulation container 9b.

In an alternative embodiment, not shown, the membrane is arranged dividing the accumulation containers 9b, 9c, 9b', 9c' along any arbitrary direction e.g. substantially along a horisontal plane.

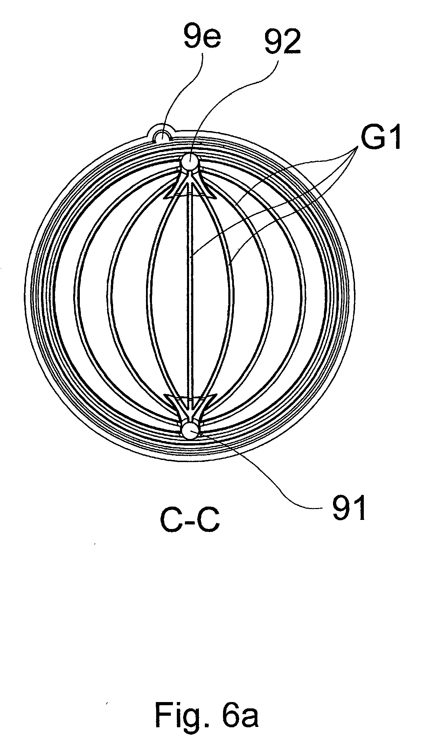

Figure 6a shows a rigid wall of a first accumulation chamber 9b according to Figure 4b provided with a plurality of grooves G1. All grooves extends between the inlet opening 91 and the outlet opening 92 such that when the flexible member 9a is rested against the rigid wall 9g of the first accumulation container 9b a continuous, although during certain periods, e.g. phase shift varying, blood flow is provided for. In Figure 6a is also shown a notch 9e provided to receive a corresponding extension, provided for orientation purposes, on the flexible member 9a.

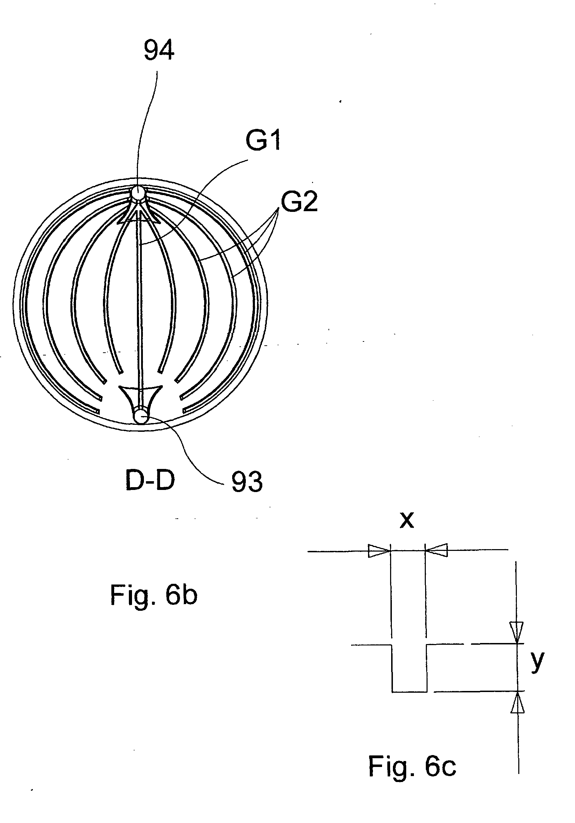

Figure 6b shows a rigid wall 9f of a second accumulation chamber 9c according to Figure 4 provided with a first groove G1 that extends between an inlet opening 93 and an outlet opening 94 and a plurality of grooves G2 that extends from an area surrounding the inlet opening 93 and the outlet opening 94. The plurality of grooves G2 will be disconnected from the inlet opening 93 when the flexible member 9a has reached its end of stroke. This design provides for a continuous base flow Q through the fluid path provided by the first groove G1 also when the flexible member 9a is rested against the rigid wall 9f of the second accumulation container 9c. After closure of the inlet opening 93 the remaining blood treatment fluid is sucked out of the second accumulation container 9c, via the plurality of grooves G2, by means of the second fluid pump 15 which is run until a predefined pressure increase rate is detected.

In Figure 6c is schematically shown a cross sectional view of the first groove G1 arranged in the wall of the second accumulation container 9c, 9c', having a width X and a depth Y. In one embodiment of the invention the cross sectional area A1, where A1=X·Y, of the first groove G is less than the cross sectional area of the respective inlet and outlet openings 93-94. In one example of the embodiment the ratio between the cross sectional area of the first groove G1 and e.g. the cross sectional area of the inlet is substantially 1/10. In another embodiment of the present invention the ratio between the width X and the depth Y of the first groove G1 is less than 1, i.e. X/Y<1. In one example of the embodiment the ratio between the width X and the depth Y of the first groove G1 is substantially 1/1,5. In one example of the embodiment the ratio between the cross sectional area of the first groove G1 and e.g. the cross sectional area of the inlet is substantially 1/10 and the ratio between the width X and the depth Y of the first groove G1 is substantially 1/1,5.

At a predefined pressure increase rate monitored by the pressure measuring means it is concluded that both flexible members 9a, 9a' have reached their end of stroke. When the end of stroke is concluded the system switches to the next phase in the cyclic process.

In an alternative embodiment of the invention end of stroke of both flexible members 9a, 9a' is concluded based on a certain volume of blood treatment fluid F being pumped. The conclusion of end of stroke of the flexible member 9a; 9a' is useful in the process of balancing and synchronizing the blood treatment apparatus comprising the disclosed membrane pumps 1a, 1b. The balancing and synchronizing of the blood treatment apparatus is optionally accomplished by means of adjustment of the base flow Q.

According to one embodiment of the invention the flexible member 9a, 9a' has a flexibility that allows unaffected transfer of pressure from the first to the second accumulation container and avoids stretching of the flexible member 9a; 9a' to an extent that it jams any of the grooves G1, G2. In one embodiment of the invention the flexible member 9a, 9a' may be made from any elastomer or rubber e.g. PVC, PUR, silicone or a polyolefin mixture. The flexible member 9a, 9a' may be manufactured by means of injection-molding. A flexible member 9a; 9a' being molded of silicone rubber is flexed rather than stretched during operation.

The membrane pump may in one embodiment be made in a material that allows sterilization by e.g. steam, heat and ethylene oxide or gamma or beta radiation.

The exemplified embodiments refer to a first needle N1 being connected via a first needle connector 31 to a first blood conduit 530 and to a blood source S and a second needle N2 being connected via a second needle connector 32 to a second blood conduit 540 and to a target vessel T. However, in an alternative embodiment the first and the second blood conduits 530, 540 may be connected to a single needle connector (not shown) that in its turn is connected to a patient via a single needle.

The term "comprises/comprising" when used in this specification is taken to specify the presence of stated features, integers, steps or components. However, the term does not preclude the presence or addition of one or more additional features, integers, steps or components or groups thereof.

The reference to prior art in this specification is not, and should not be taken as, an acknowledgment or any suggestion that the referenced prior art forms part of the common general knowledge in Australia, or in any other country.

## Claims

1. A membrane pump comprising a pumping chamber and a flexible member (9a, 9a') separating the pumping chamber into a first accumulation container (9b, 9b') and a second accumulation container (9c, 9c'), the flexible member (9a, 9a') being configured to be movable within the pumping chamber so as to vary a volume relationship between the first and second accumulation container (9b, 9b', 9c, 9c'), wherein the flexible member is provided with a rim (9d) around its circumference such that it is mounted between rigid walls of the first and second accumulation containers (9b, 9b', 9c, 9c'), the second accumulation container (9c, 9c'), being configured to receive an amount of working fluid (F) to act on the flexible member (9a, 9a') and thus, in use, pump a first fluid (B) from the first accumulation container (9b, 9b'), and the first and the second accumulation container (9b,9c; 9b',9c'), each has an inlet opening (91, 93) for inlet of the respective fluid (B, F) and an outlet opening (92, 94) for outlet of the respective fluid (B, F), the inlet opening (91, 93) being arranged on one side of the accumulation container (9b, 9b', 9c, 9c') and the outlet opening being arranged on the opposite side of the accumulation container (9b, 9c; 9b',9c'), **characterized in that** the flexible member (9a, 9a') is configured to show, when arranged in the pumping chamber and seen in a cross section along a plane extending through a pair of the inlet and outlet openings (91, 92; 93, 94) of the accumulation container (9b,9c; 9b', 9c'), a thickness that is increased in a downstream direction such that it provides a reinforced portion in the area of the respective outlet openings (92, 94).

2. The membrane pump according to claim 1 wherein the thickness of the flexible member (9a, 9a') is increased in a continuous manner.

3. The membrane pump according to claim 1 or 2 wherein the thickness of the flexible member (9a, 9a') is increased by at least 50%.

4. The membrane pump according to claim 1, 2 or 3 wherein at least a portion of the flexible member (9a, 9a') has a dome shape.

5. The membrane pump according to claim 4 wherein the dome shaped portion is formed as a section of a spherical object having an inner radius R1 and an outer radius R2 where the central point for the outer radius is offset with respect to the central point of the inner radius.

6. The membrane pump according to claim 1 wherein a rigid wall of the second accumulation container (9c, 9c') is provided with at least one groove (G1) extending between the inlet (93) and the outlet (94) where the at least one groove (G1) has a cross sectional area that is less than the cross sectional area of the inlet opening (93).

7. The membrane pump according to claim 6 wherein the at least one groove (G1) has a cross sectional area that is substantially 1/10 of the cross sectional area of the inlet opening (93).

8. The membrane pump according to claim 6 or 7 wherein the at least one groove (G1) has a width (X) and a depth (Y) where the ratio between the width (X) and the depth (Y) is <1.

9. The membrane pump according to claim 8 wherein the ratio between the width (X) and the depth (Y) is substantially 1/1,5.

10. The membrane pump according to claim 6 wherein the rigid wall of the second accumulation container (9c, 9c') is provided with a plurality of grooves (G2) extending between an area around the inlet (93) and the outlet (94) such that, upon closure of the inlet (93) by means of the flexible member (9a, 9a'), a flow path disconnected from the inlet (93) is provided.

11. The membrane pump according to claim 1 wherein, in use, the flexible member (9a, 9a') is arranged dividing the first and the second accumulation containers (9b, 9b', 9c, 9c') along a substantially vertical plane.

12. A blood treatment apparatus, comprising:
- a blood treatment unit (8) configured to receive untreated blood and fresh blood treatment fluid, and emit treated blood and used blood treatment fluid, connectable to
- at least one fluid pump (14, 15) configured to pass blood treatment fluid through the blood treatment unit (8), and
- at least two blood pumps (1a, 1b) configured to extract untreated blood from a blood source (P, S), pass extracted blood through the blood treatment unit (8) and deliver treated blood to a target vessel (P, T), **characterized in that**
- at least one of the at least one fluid pump (14, 15) is configured to control the operation of the blood pumps (1a, 1b) via the blood treatment fluid, and
- the blood pumps (1a, 1b) are integrated into a joint apparatus element (D) comprising the blood treatment unit (8) and are membrane pumps as claimed in any of claims 1-11.

13. The blood treatment apparatus according to claim 12, wherein each of the at least one blood pump (1a, 1b) is configured to be in fluid connection with the treatment unit (8) throughout an entire treatment of an amount of blood from the blood source (S).

14. The blood treatment apparatus according to claim 13, comprising:
- a first blood conduit (31, 530) configured to be connected to the blood source (S),
- a second blood conduit (32, 540) configured to be connected to the target vessel (T), and
- at least one needle connector (31, 32) configured to connect the first and second blood conduits (530, 540) with at least one needle (N1, N2).

15. The blood treatment apparatus according to claim 14, wherein
- the joint apparatus element (D) comprises a body module having an essentially cylindrical outline with a central length axis, and
- at least one of the at least one blood pump (1a, 1b) in the joint apparatus element (D) is arranged on a side surface of the blood treatment unit (8) such that the at least one blood pump (1a, 1b) is arranged essentially asymmetrically with respect to the central length axis.

## Patentansprüche

1. Membranpumpe, umfassend eine Pumpkammer und ein flexibles Element (9a, 9a'), das die Pumpkammer in einen ersten Sammelbehälter (9b, 9b') und einen zweiten Sammelbehälter (9c, 9c') aufteilt, wobei das flexible Element (9a, 9a') so eingerichtet ist, dass es innerhalb der Pumpkammer beweglich ist, um ein Volumenverhältnis zwischen dem ersten und zweiten Sammelbehälter (9b, 9b', 9c, 9c') zu verändern, wobei das flexible Element mit einem Rand (9d) um seinen Umfang derart versehen ist, dass es zwischen starren Wänden des ersten und zweiten Sammelbehälters (9b, 9b', 9c, 9c') gefasst ist, wobei der zweite Sammelbehälter (9c, 9c') so eingerichtet ist, dass er eine Menge Arbeitsfluid (F) aufnimmt, das auf das flexible Element (9a, 9a') einwirkt und so, während des Einsatzes, ein erstes Fluid (B) aus dem ersten Sammelbehälter (9b, 9b') pumpt, und wobei der erste und der zweite Sammelbehälter (9b, 9c; 9b', 9c') jeweils eine Einlassöffnung (91, 93) für die Zuleitung des jeweiligen Fluids (B, F) und eine Auslassöffnung (92, 94) für die Ableitung des jeweiligen Fluids (B, F) aufweisen, wobei die Einlassöffnung (91, 93) auf einer Seite des Sammelbehälters (9b, 9b', 9c, 9c') angeordnet ist und die Auslassöffnung auf der gegenüberliegenden Seite des Sammelbehälters (9b, 9c; 9b', 9c') angeordnet ist, **dadurch gekennzeichnet, dass** das flexible Element (9a, 9a') so eingerichtet ist, dass, wenn es in der Pumpkammer angeordnet ist und in einem Querschnitt entlang einer Ebene betrachtet wird, die durch ein Paar aus Einlass- und Auslassöffnung (91, 92; 93, 94) des Sammelbehälters (9b, 9c; 9b', 9c') verläuft, es eine Dicke aufweist, die in einer Stromrichtung zunimmt, sodass es einen verstärkten Abschnitt im Bereich der jeweiligen Auslassöffnungen (92, 94) vorsieht.

2. Membranpumpe nach Anspruch 1, wobei die Dicke des flexiblen Elements (9a, 9a') kontinuierlich zunimmt.

3. Membranpumpe nach Anspruch 1 oder 2, wobei die Dicke des flexiblen Elements (9a, 9a') um mindestens 50 % zunimmt.

4. Membranpumpe nach Anspruch 1, 2 oder 3, wobei zumindest ein Abschnitt des flexiblen Elements (9a, 9a') eine Kuppelform aufweist.

5. Membranpumpe nach Anspruch 4, wobei der kuppelförmige Abschnitt als ein Teilstück eines kugelförmigen Gegenstands mit einem Innenradius R1 und einem Außenradius R2 geformt ist, bei dem der Mittelpunkt für den Außenradius bezogen auf den Mittelpunkt des Innenradius versetzt ist.

6. Membranpumpe nach Anspruch 1, wobei eine starre Wand des zweiten Sammelbehälters (9c, 9c') mit mindestens einer Rille (G1) versehen ist, die zwischen dem Einlass (93) und dem Auslass (94) verläuft, wobei die mindestens eine Rille (G1) eine Querschnittsfläche aufweist, die geringer als die Querschnittsfläche der Einlassöffnung (93) ist.

7. Membranpumpe nach Anspruch 6, wobei die mindestens eine Rille (G1) eine Querschnittsfläche aufweist, die im Wesentlichen 1/10 der Querschnittsfläche der Einlassöffnung (93) beträgt.

8. Membranpumpe nach Anspruch 6 oder 7, wobei die mindestens eine Rille (G1) eine Breite (X) und eine Tiefe (Y) aufweist, wobei das Verhältnis zwischen der Breite (X) und der Tiefe (Y) <1 beträgt.

9. Membranpumpe nach Anspruch 8, wobei das Verhältnis zwischen der Breite (X) und der Tiefe (Y) im Wesentlichen 1/1,5 beträgt.

10. Membranpumpe nach Anspruch 6, wobei die starre Wand des zweiten Sammelbehälters (9c, 9c') mit einer Vielzahl von Rillen (G2) versehen ist, die zwischen einem Bereich um den Einlass (93) und den Auslass (94) derart verlaufen, dass beim Verschließen des Einlasses (93) mittels des flexiblen Elements (9a, 9a') ein von dem Einlass (93) getrennter Strömungsweg bereitgestellt ist.

11. Membranpumpe nach Anspruch 1, bei der während des Einsatzes das flexible Element (9a, 9a') so angeordnet ist, dass es den ersten und den zweiten Sammelbehälter (9b, 9b', 9c, 9c') entlang einer im Wesentlichen senkrechten Ebene unterteilt.

12. Blutbehandlungsvorrichtung, umfassend:
- eine Blutbehandlungseinheit (8), die so eingerichtet ist, dass sie unbehandeltes Blut und frisches Blutbehandlungsfluid aufnimmt und behandeltes Blut und verbrauchtes Blutbehandlungsfluid abgibt, und verbindbar ist mit
- mindestens einer Fluidpumpe (14, 15), die so eingerichtet ist, dass sie Blutbehandlungsfluid durch die Blutbehandlungseinheit (8) leitet, und
- mindestens zwei Blutpumpen (1a, 1 b), die so eingerichtet sind, dass sie unbehandeltes Blut aus einer Blutquelle (P, S) entnehmen, entnommenes Blut durch die Blutbehandlungseinheit (8) leiten und behandeltes Blut an ein Zielgefäß (P, T) abgeben, **dadurch gekennzeichnet, dass**
- mindestens eine von der mindestens einen Fluidpumpe (14, 15) so eingerichtet ist, dass sie den Betrieb der Blutpumpen (1 a, 1 b) über das Blutbehandlungsfluid steuert, und
- die Blutpumpen (1a, 1 b) in ein gemeinsames Vorrichtungselement (D) integriert sind, das die Blutbehandlungseinheit (8) umfasst, und Membranpumpen nach einem der Ansprüche 1 bis 11 sind.

13. Blutbehandlungsvorrichtung nach Anspruch 12, wobei jede von der mindestens einen Blutpumpe (1 a, 1 b) so eingerichtet ist, dass sie mit der Behandlungseinheit (8) während einer kompletten Behandlung einer Blutmenge von der Blutquelle (S) in Fluidverbindung steht.

14. Blutbehandlungsvorrichtung nach Anspruch 13, umfassend:
- einen ersten Blutschlauch (31, 530), der so eingerichtet ist, dass er mit der Blutquelle (S) verbunden wird,
- einen zweiten Blutschlauch (32, 540), der so eingerichtet ist, dass er mit dem Zielgefäß (T) verbunden wird, und
- mindestens ein Nadelverbindungselement (31, 32), das so eingerichtet ist, dass es den ersten und zweiten Blutschlauch (530, 540) mit mindestens einer Nadel (N1, N2) verbindet.

15. Blutbehandlungsvorrichtung nach Anspruch 14, wobei
- das gemeinsame Vorrichtungselement (D) ein Körpermodul umfasst, das einen im Wesentlichen zylindrischen Umriss mit einer mittigen Längsachse aufweist, und
- mindestens eine von der mindestens einen Blutpumpe (1 a, 1 b) in dem gemeinsamen Vorrichtungselement (D) an einer Seitenfläche der Blutbehandlungseinheit (8) derart angeordnet ist, dass die mindestens eine Blutpumpe (1 a, 1 b) im Wesentlichen asymmetrisch bezogen auf die mittige Längsachse angeordnet ist.

## Revendications

1. Pompe à membrane comprenant une chambre de pompage et un élément flexible (9a, 9a') divisant la chambre de pompage en un premier réservoir d'accumulation (9b, 9b') et un second réservoir d'accumulation (9c, 9c'), l'élément flexible (9a, 9a') étant configuré pour être mobile dans la chambre de pompage de manière à varier un rapport de volume entre les premier et second réservoirs d'accumulation (9b, 9b', 9c, 9c'), dans laquelle l'élément flexible est doté d'un bord (9d) autour de sa circonférence de sorte qu'il est monté entre des parois rigides des premier et second réservoirs d'accumulation (9b, 9b', 9c, 9c'), le second réservoir d'accumulation (9c, 9c') étant configuré pour recevoir une quantité de fluide de travail (F) pour agir sur l'élément flexible (9a, 9a') et ainsi, lors de l'utilisation, pomper un premier fluide (B) du premier réservoir d'accumulation (9b, 9b'), et les premier et second réservoirs d'accumulation (9b, 9c ; 9b', 9c') présentant chacun une ouverture d'entrée (91, 93) pour l'entrée du fluide respectif (B, F) et une ouverture de sortie (92, 94) pour la sortie du fluide respectif (B, F), l'ouverture d'entrée (91, 93) étant agencée d'un côté du réservoir d'accumulation (9b, 9b', 9c, 9c') et l'ouverture de sortie étant agencée du côté opposé du réservoir d'accumulation (9b, 9c ; 9b', 9c'), **caractérisée en ce que** l'élément flexible (9a, 9a') est configuré pour, quand il est agencé dans la chambre de pompage et vu en coupe le long d'un plan s'étendant à travers une paire des ouvertures d'entrée et de sortie (91, 92 ; 93, 94) du réservoir d'accumulation (9b, 9c ; 9b', 9c'), présenter une épaisseur qui est augmentée dans une direction d'écoulement de sorte qu'une partie renforcée est fournie dans la zone des ouvertures de sortie respectives (92, 94).

2. Pompe à membrane selon la revendication 1, dans laquelle l'épaisseur de l'élément flexible (9a, 9a') est augmentée de manière continue.

3. Pompe à membrane selon la revendication 1 ou 2, dans laquelle l'épaisseur de l'élément flexible (9a, 9a') est augmentée d'au moins 50 %.

4. Pompe à membrane selon la revendication 1, 2 ou 3, dans laquelle au moins une partie de l'élément flexible (9a, 9a') présente une forme de dôme.

5. Pompe à membrane selon la revendication 4, dans laquelle la partie en forme de dôme est formée comme section d'un objet sphérique présentant un rayon intérieur R1 et un rayon extérieur R2, le point central pour le rayon extérieur étant décalé par rapport au point central du rayon intérieur.

6. Pompe à membrane selon la revendication 1, dans laquelle une paroi rigide du second réservoir d'accumulation (9c, 9c') est dotée d'au moins une rainure (G1) s'étendant entre l'entrée (93) et la sortie (94), l'au moins une rainure (G1) présentant une superficie en coupe qui est inférieure à la superficie en coupe de l'ouverture d'entrée (93).

7. Pompe à membrane selon la revendication 6, dans laquelle l'au moins une rainure (G1) présente une superficie en coupe qui est essentiellement 1 /10 de la superficie en coupe de l'ouverture d'entrée (93).

8. Pompe à membrane selon la revendication 6 ou 7, dans laquelle l'au moins une rainure (G1) présente une largeur (X) et une profondeur (Y), le rapport entre la largeur (X) et la profondeur (Y) étant <1.

9. Pompe à membrane selon la revendication 8, dans laquelle le rapport entre la largeur (X) et la profondeur (Y) est essentiellement d'1 /1,5.

10. Pompe à membrane selon la revendication 6, dans laquelle la paroi rigide du second réservoir d'accumulation (9c, 9c') est dotée d'une pluralité de rainures (G2) s'étendant entre une zone autour de l'entrée (93) et la sortie (94) de sorte que, lors de la fermeture de l'entrée (93) au moyen de l'élément flexible (9a, 9a'), une voie d'écoulement séparée de l'entrée (93) est réalisée.

11. Pompe à membrane selon la revendication 1, dans laquelle, lors de l'utilisation, l'élément flexible (9a, 9a') est agencé divisant les premier et second réservoirs d'accumulation (9b, 9b', 9c, 9c') le long d'un plan essentiellement vertical.

12. Appareil de traitement du sang, comprenant :
- une unité de traitement du sang (8) configurée pour recevoir du sang non traité et du fluide de traitement du sang frais, et émettre du sang traité et du fluide de traitement du sang usagé, pouvant être raccordée à
- au moins une pompe à fluide (14, 15) configurée pour faire passer du fluide de traitement du sang à travers l'unité de traitement du sang (8), et
- au moins deux pompes à sang (1 a, 1 b) configurées pour extraire du sang non traité d'une source de sang (P, S), faire passer du sang extrait à travers l'unité de traitement du sang (8) et délivrer du sang traité dans un vaisseau cible (P, T), **caractérisé en ce que**
- au moins l'une de l'au moins une pompe à fluide (14, 15) est configurée pour commander le fonctionnement des pompes à sang (1 a, 1 b) par l'intermédiaire du fluide de traitement du sang, et
- les pompes à sang (1a, 1 b) sont intégrées dans un élément d'appareil commun (D) comprenant l'unité de traitement du sang (8) et sont des pompes à membrane selon l'une quelconque des revendications 1 à 11.

13. Appareil de traitement du sang selon la revendication 12, dans lequel chacune de l'au moins une pompe à sang (1 a, 1 b) est configurée pour être en liaison fluidique avec l'unité de traitement (8) pendant un traitement entier d'une quantité de sang venant de la source de sang (S).

14. Appareil de traitement du sang selon la revendication 13, comprenant :
- un premier conduit de sang (31, 530) configuré pour être raccordé à la source de sang (S),
- un second conduit de sang (32, 540) configuré pour être raccordé au vaisseau cible (T), et
- au moins un connecteur d'aiguille (31, 32) configuré pour raccorder les premier et second conduits de sang (530, 540) à au moins une aiguille (N1, N2).

15. Appareil de traitement du sang selon la revendication 14, dans lequel
- l'élément d'appareil commun (D) comprend un module de corps présentant un contour essentiellement cylindrique avec un axe longitudinal central, et
- au moins l'une de l'au moins une pompe à sang (1 a, 1 b) dans l'élément d'appareil commun (D) est agencée sur une surface latérale de l'unité de traitement du sang (8) de sorte que l'au moins une pompe à sang (1 a, 1 b) est agencée essentiellement de manière asymétrique par rapport à l'axe longitudinal central.
